# EUROPEAN PATENT SPECIFICATION

(11) **EP 1 784 156 B1**
(45) Date of publication and mention of the grant of the patent: **07.12.2011**
(21) Application number: 05787285.5
(22) Date of filing: 29.08.2005
(51) Int. Cl.: A61K 8/34, A61Q 17/04

(54) **MICRO-PARTICULATE ORGANIC UV ABSORBER COMPOSITION**
MIKROPARTIKULÄRE, ORGANISCHE, UV-ABSORBIERENDE ZUSAMMENSETZUNG
COMPOSITION D'ABSORBEUR D'UV ORGANIQUE A MICROPARTICULES

(30) Priority: 01.09.2004 EP 04104185
(43) Date of publication of application: 16.05.2007
(73) Proprietor: BASF SE, 67056 Ludwigshafen (DE)
(72) Inventor: DESHAYES, Cyrille, F-68128 Rosenau (FR); MONGIAT, Sébastien, F-68510 Sierentz (FR)
(86) International application number: PCT/EP2005/054225
(87) International publication number: WO 2006/024633

(56) References cited:
- EP-A- 1 068 866
- EP-A- 1 285 648
- WO-A-03/063814
- WO-A-03/070202
- US-B1- 6 521 217
- US-B1- 6 746 666
- DATABASE CHEMABS CHEMICAL ABSTRACTS SERVICE, COLUMBUS, OHIO, US; XP002322968 retrieved from STN Database accession no. 140: 275 747 & JP 2004 091374 A (SHISEIDO CO., LTD) 25 March 2004 (2004-03-25)

## Description

The present invention relates to topically applicable water-in-oil (w/o) or water-in-silicone (w/Si) formulations, in particular, to new UV-protection formulations which comprise a micronised UV absorber, wherein the micronised UV absorber is combined with specific fatty acid alcohol.

It has long been known that prolonged exposure to UV radiation can lead to the formation of erythemas or light dermatoses, as well as to an increased incidence of skin cancers, or accelerated skin ageing.

Various sunscreen formulations have been proposed which include materials which are intended to counteract UV radiation, thereby inhibiting the said undesired effects on the skin.

A great number of compounds have been proposed for use as UV protectants in sunscreen formulations, especially soluble organic UV absorbers and insoluble micronised inorganic compounds, in particular zinc oxide and titanium dioxide.

With respect to the use in sunscreen formulations of soluble organic UV absorbers, they have the disadvantages that their effectiveness as UV protectants in terms of SPF (Sun Protection Factor) in a sunscreen formulation is often too low for commercial purposes; as a result of their solubility, they exhibit relatively high allergenic potential; and that as a result of intrinsic photochemical lability, the duration of the protective effect is often too low.

The high specific weight of insoluble inorganic compounds, such as titanium dioxide leads to a reduced stability of formulations containing them. Moreover, such inorganic compounds have been claimed to generate toxic radicals under the influence of light and water ("Redox Mechanisms in Heterogeneous Photocatalysis", Serpone et al, Electrochemistry in Colloids and Dispersions, Editors Mackay and Texter, VCH Publishers Inc., NewYork 1992).

In GB-A-2303549, there is described a method of producing micronised, insoluble organic UV absorbers, as well as a sunscreen composition comprising a micronised formulation of an insoluble organic UV absorber, produced according to the said method.

Micronised insoluble organic When the so obtained UV absorbers are used in sunscreen formulations theyprovide excellent UV protection and have an SPF rating which is at least as high as corresponding sunscreen formulations containing a known inorganic UV absorber. Unlike the latter UV absorbers micronised insoluble organic UV absorbers show no tendency for generating radicals which could damage or sensitise human skin under the influence of light.

In WO 99/66896 a micronised organic UV absorber sun screen composition is disclosed, wherein the micronised organic UV absorber is present in the oil phase of the formulation.

US 6521,217 discloses sunscreen formulations, comprising micronized organic UV absorbers and a cosmetically acceptable carrier, wherein the micronized organic UV absorber is present in the oil phase. Example 5 discloses a W/O emulsion comprising 1,2 g of Methylene Bis-Benzotriazolyl Tetramethylbutylphenol (Tinosorb M) and 6 g Octyldodecanol.

EP 1 068 866 discloses the use of mixtures of micropigments for tanning prevention and lightening of skin and hair. Example 38 discloses a W/O emulsion comprising Octyldodecanol and a micropigment mixture comprising Methylene Bis-Benzotriazolyl Tetramethylbutvlphenol (Tinosorb M).

US 6,746,666 discloses the use of mixtures of micronized organic UV filters like Methylene Bis-Benzotriazolyl Tetramethylbutylphenol (Tinosorb M) in protecting human and animal skin and hair against the damaging effect of UV radiation.

WO 03/063814 discloses UV absorber mixtures comprising a micronized UV broadband filter like Methylene Bis-Benzotriazolvl Tetramethylbutylphenol (Tinosorb M) and a water dispersible and/or oil dispersable coated titanium dioxide. On page 29 a generic W/O formulation with 10-15 % b.w. of a fatty alcohol and 1-20 % b.w. of micronized UV filters is disclosed.

CA 140:275747 discloses water-in-silicone sunscreens comprising Methylene Bis-Benzotriazolyl Tetramethylbutylphenol (Tinosorb M) and polyols.

WO 03/070202 discloses biphasic sunscreen compositions comprising a W/O emulsion, a lipophilic or aqueous phase, at least one W/O emulsifier and at least one organic and/or inorganic UV filter, wherein at least one phase is not transparent or translucent. Example 1, column 3 discloses a W/O sunscreen comprising Methylene Bis-Benzotriazolyl Tetramethylbutylphenol (Tinosorb M) and optionally an alcohol which is present in the outer phase.

EP 1 285 648 discloses sunscreens comprising an oil soluble UV filter and imido succinic acid. Example 3. col. 3 discloses a W/O sunscreen comprising Methylene Bis-Benzotriazolyl Tetramethylbutylphenol (Tinosorb M) and and optionally an alcohol which is present in the outer phase.

Surprisingly it was found that the combination of a micronized organic UV absorber and a specific fatty alcohol allows a maximum of miscibility of micronized organic particles within the continuous oil phase of w/o emulsions wherein the micronized particles are stabilized in the oil phase of the composition. Simultaneously the crystal growth of micronized organic UV absorbers within W/O emulsions is limited or even eliminated.

Therefore, the present invention relates to a topically applicable water-in-oil (w/o) or water-in-silicone (w/Si) formulation comprising:
(a) 10 to 90 % b.w. of at least one aqueous phase;
(b) 1 to 60 % b.w. of at least one fatty phase;
(c) 0.1 to 30 % b.w. of an effective UV-photoprotecting composition comprising
   (c₁) 20% to 99% of at least one insoluble micronized organic UV-screening active with a mean particle size ranging from 0,01 µm to 2 µm;
   (c₂) 0.5% to 80% of a mixture of linear octanol and linear decanol;
      0 to 30 % b.w. of at least one emulsifier; and
      0 to 88.9 % b.w. of further cosmetically acceptable adjuvants.

Suitable organic UV absorber (c₁) may be, e.g. a triazine, a benzotriazole, a benzophenone, a vinyl group-containing amide, a cinnamic acid amide or a sulfonated benzimidazole UV absorber.

A preferred class of triazine compounds is that having the formula 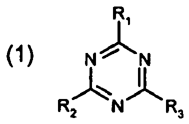 , wherein
R₁, R₂ and R₃, independently from each other, are hydrogen; hydroxy; C₁-C₃alkoxy; NH₂; NHR₄; N(R₄)₂; OR₄; C₆-C₁₂aryl; phenoxy; anilino; pyrrolo; in which the respective phenyl, phenoxy, anilino or pyrrolo moieties are not substituted or substituted by one, two or three substitutents selected from OH, carboxy, CO-NH₂, C₁-C₁₂alkyl, C₁-C₁₂alkoxy, a methylidenecamphor group, a group -(CH=CH)ₘC(=O)-OR₄, a group 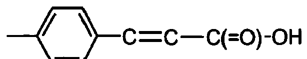 or the corresponding alkali metal, ammonium, mono-, di- or tri-C₁-C₄alkylammonium, mono-, di- or tri-C₂-C₄alkanolammonium salts, or the C₁-C₃alkyl esters thereof or by a radical of formula 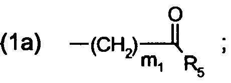 R₄ is C₁-C₅alkyl;
R₅ is hydroxy; C₁-C₅alkyl that is unsubstituted or substituted by one or more OH groups; C₁-C₅alkoxy; amino; mono- or di-C₁-C₅alkylamino; M; a radical of formula 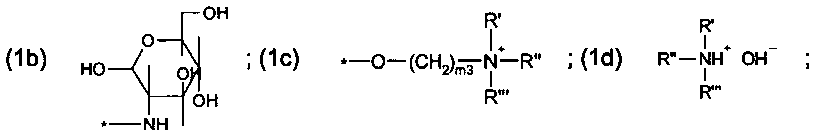 or 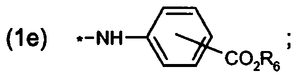 R', R" and R"' independently of the other are C₁-C₁₄alkyl that is unsubstituted or substituted by one or more OH groups;
R₆ is hydrogen; M; C₁-C₅alkyl; or a radical of the formula 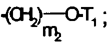 M is a metal cation;
T₁ is hydrogen; or C₁-C₈alkyl;
m is 0 or 1;
m₁ is from 1 to 5;
m₂ is from 1 to 4; and
m₃ is from 2 to 14.

Preferred compounds of formula (1) are those, wherein
R₁, R₂ and R₃ independently from each other are a radical of formula 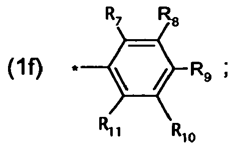 or 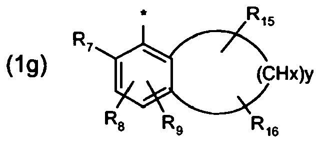 R₇ and R₁₁ independently from each other are hydrogen; C₁-C₁₈alkyl; or C₆-C₁₂aryl;
R₈, R₉ and R₁₀, independently from each other, are hydrogen; or a radical of formula 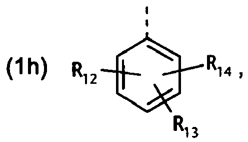 wherein, in formula (1f), at least one of the radicals R₈, R₉ and R₁₀ are a radical of formula (1h);
R₁₂, R₁₃, R₁₄, R₁₅ and R₁₆ independently from each other are hydrogen; hydroxy; halogen; C₁-C₁₈alkyl; C₁-C₁₈alkoxy; C₆-C₁₂aryl; biphenylyl; C₆-C1₂aryloxy; C₁-C₁₈alkylthio; carboxy; -COOM; C₁-C₁₈-alkylcarboxyl; aminocarbonyl; or mono- or di-C₁-C₁₈alkylamino; C₁-C₁₀acylamino; -COOH;
M is an alkali metal ion;
x is 1 or 2; and
y is a number from 2 to 10.

Most preferred triazine derivatives are compounds of formula 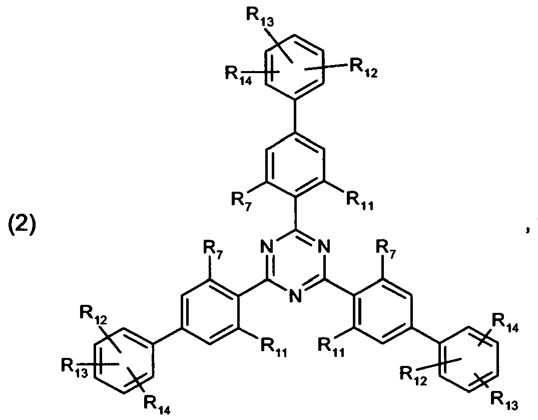 wherein
R₇, R₁₁, R₁₂, R₁₃ und R₁₄ are defined as in formula (1f), (1g) or (1h), and most preferably compound of formula (2), wherein
R₇ and R₁₁ are hydrogen.

Furthermore, triazinederivatives of formula 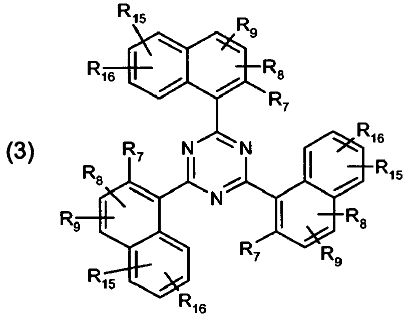 are preferred, wherein
R₇, R₈, R₉, R₁₅ and R₁₆ are defined as in formula (1g), and most preferably compounds of formula (3), wherein
R₇, R₈, R₉, R₁₅ and R₁₆ are hydrogen; or, independently from each other, C₁-C₁₆alkyl.

Most preferred as component (c₁) are triazine derivatives of formula

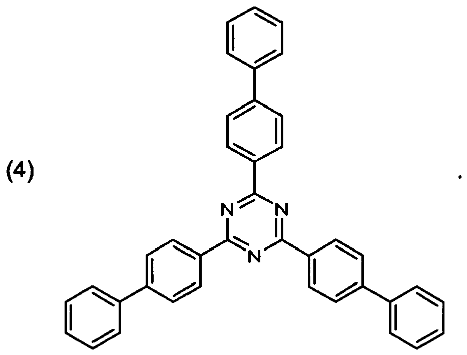

Further preferred triazine derivatives according to component (c₁) correspond to formula 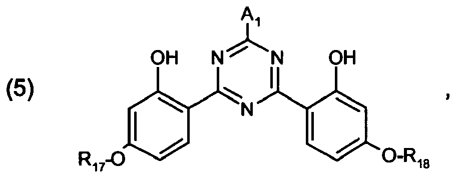 wherein
R₁₇ and R₁₈, independently of one another, are C₁-C₁₈alkyl; C₂-C₁₈alkenyl; a radical of the formula -CH₂-CH(-OH)-CH₂-O-T₁ ; or a radical of the formula 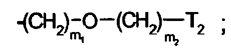 a radical of the formula 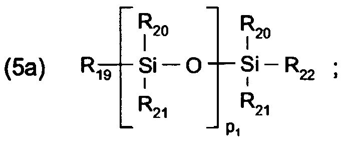 R₁₉ is a direct bond; a straight-chain or branched C₁-C₄alkylene radical or a radical of the formula 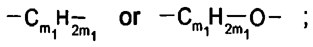 R₂₀, R₂₁ and R₂₂, independently of one another, are C₁-C₁₈alkyl; C₁-C₁₈alkoxy or a radical of the formula 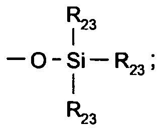 R₂₃ is C₁-C₅alkyl;
T₁ and T₂, independently from each other, are hydrogen; or C₁-C₈alkyl;
m₁, m₂ and m₃, independently of one another, are 1 to 4;
p₁ is 0; or a number from 1 to 5;
A₁ is a radical of the formula 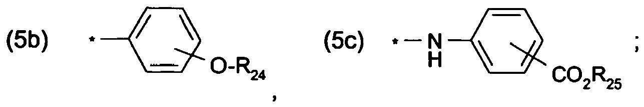 or of the formula 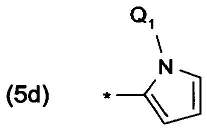 R₂₄ is hydrogen; C₁-C₁₀alkyl, -(CH₂CHR₂₆-O)_{n₁}R₂₅; a -CH₂-CH(-OH)-CH₂-O-T₁; or radical of the formula 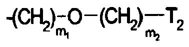 R₂₅ is hydrogen; M; C₁-C₅alkyl; or a radical of the formula -(CH₂)_{m₂}-O-T₁ ;
R₂₆ is hydrogen; or methyl;
Q₁ C₁-C₁₈alkyl;
M is a metal cation; and
n₁ is 1-16.

Further preferred triazine derivatives according to component (c₁) are compounds of formulae 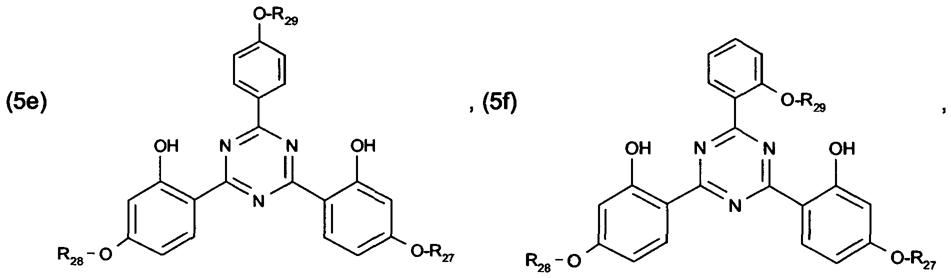 the formula 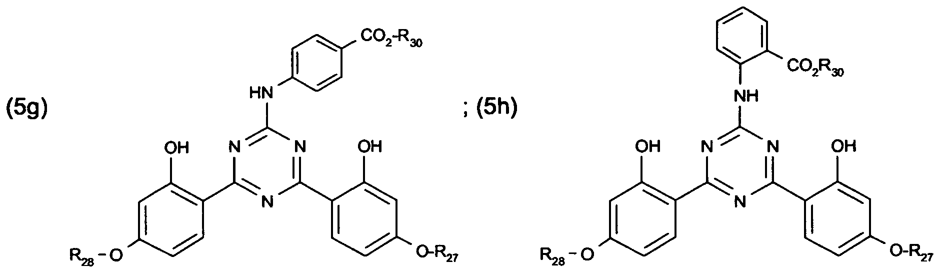 in which
R₂₇ and R₂₈, independently of one another, are C₃-C₁₈alkyl; or -CH₂CH(-OH)-CH₂-O-T₁;
R₃₀ is C₁-C₁₀alkyl or a radical of the formula 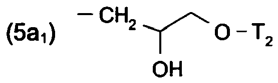 or the formula 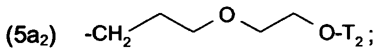 R₃₀ is hydrogen; M; C₁-C₅alkyl; -NH-C₁-C₅alkyl, preferably -NH-tert.alkyl; or a radical of the formula -(CH₂)ₘ-O-T₂;
T₁ and T₂, independently of one another, are hydrogen; or C₁-C₅alkyl; and
m is 1 to 4.

Uppermost of interest are compounds of the formulae (5e) and (5f), in which
R₂₇ and R₂₈, independently of one another, are C₃-C₁₈alkyl; or -CH₂-CH(-OH)-CH₂-O-T₁;
R₂₉ is C₁-C₁₀alkyl;
and compounds of the formulae (5g) and (5h), in which
R₂₇ and R₂₈, independently of one another, are C₃-C₁₈alkyl or -CH₂-CH(-OH)-CH₂-O-T₁; and
T₁ is hydrogen; or C₁-C₅alkyl.

Very particularly preferred in this case are triazine compounds of the formula (5e) - (5h), in which R₂₇ and R₂₈ have the same meaning.

Furthermore, interesting triazines correspond to the formula 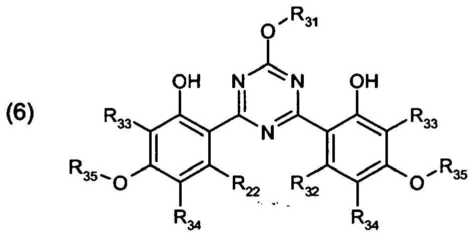 in which
R₃₁ is C₁-C₃₀alkyl; C₂-C₃₀alkenyl; unsubstituted or C₁-C₅alkyl-mono- or polysubstituted C₅-C₁₂cycloalkyl, C₁-C₅alkoxy-C₁-C₁₂alkyl; amino- C₁-C₁₂alkyl; C₁-C₅monoalkylamino-C₁-C₁₂alkyl; C₁-C₅dialkylamino-C₁-C₁₂alkyl; a radical of the formula 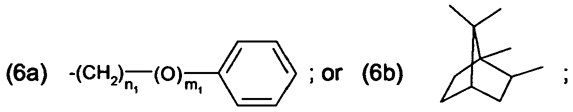 R₃₂, R₃₃ and R₃₄, independently of one another, are hydrogen;, hydroxyl; C₁-C₃₀alkyl; or C₂-C₃₀alkenyl;
R₃₅ is hydrogen; or C₁-C₅alkyl;
m₁ is 0 or 1; and
n₁ is 1 to 5.

Preferred compounds correspond to the formula 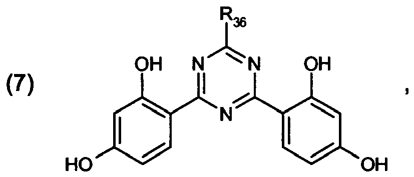 wherein
R₃₆ is 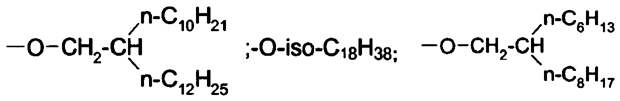 -O-n-C₁₈H₃₇; or
-O-2-ethylhexyl; -O-(CH₂)₃-N(C₂H₅)₂;_{;}

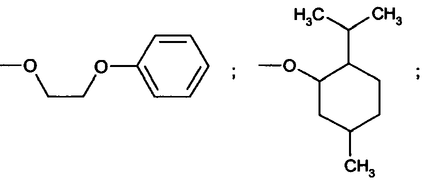

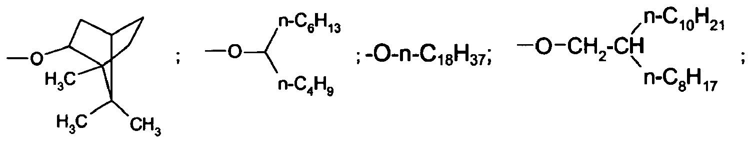

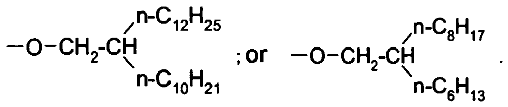

Further preferred triazine derivatives according to component (c₁) are those compounds having one of the formulae 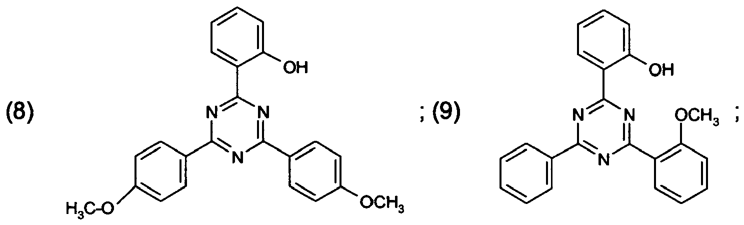 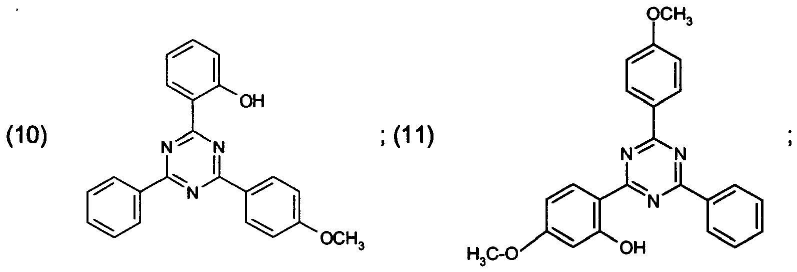 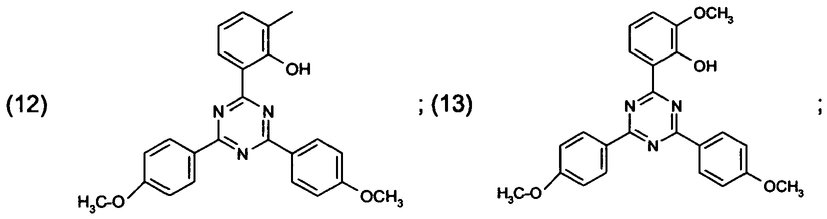 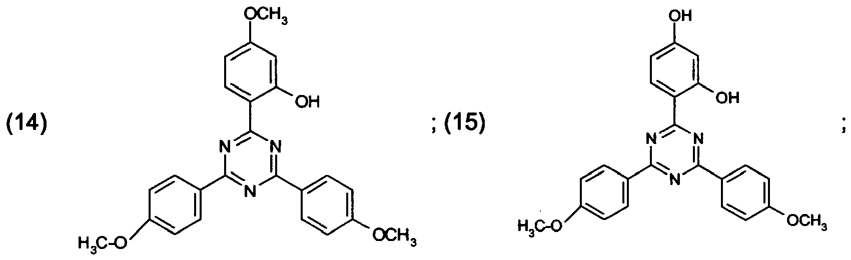 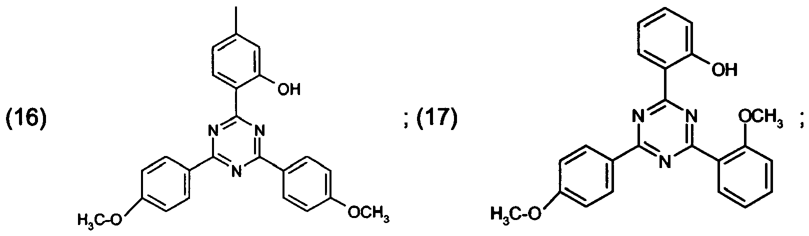 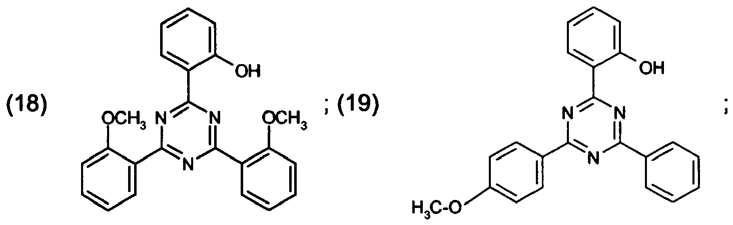 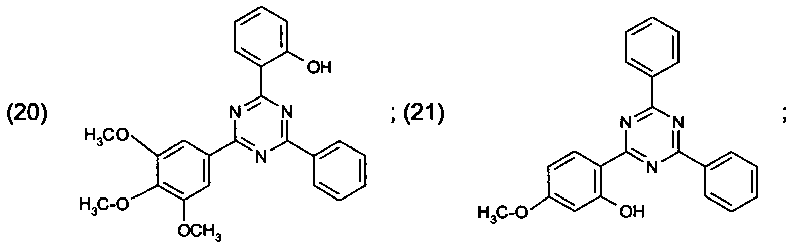 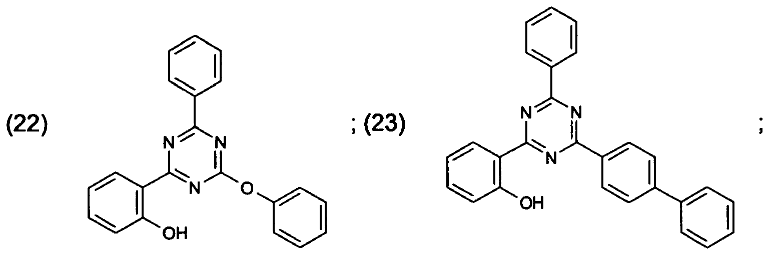 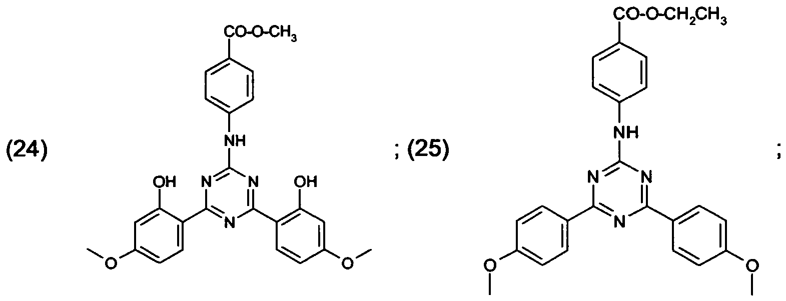 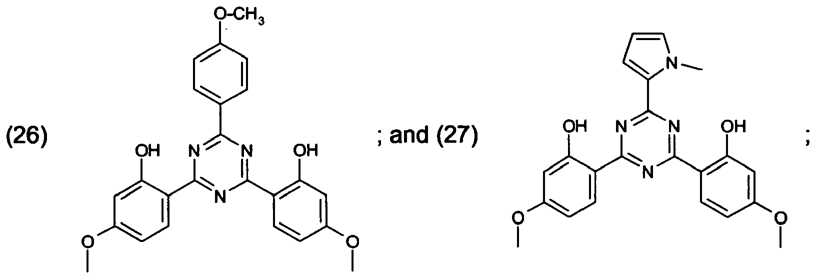 as well as 2,4,6-tris(diisobutyl-4'-aminobenzalmalonate)-s-triazine and 2,4-bis(diisobutyl-4-aminobenzalmalonate)-6-(4'-aminobenzylidenecamphor)-s-triazine.

Particularly preferred compounds of formula (1) are those having the formula: 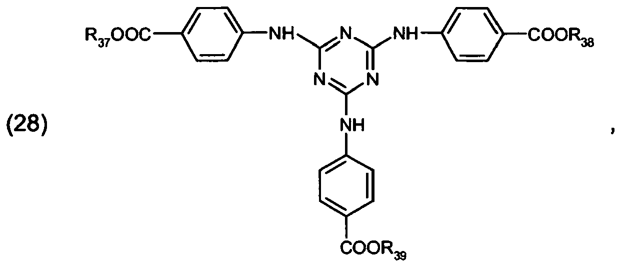 wherein
R₃₇, R₃₈ and R₃₉, independently from each other are hydrogen; an alkali metal; or an ammonium group N⁺(R₄₀)₄;
R₄₀ is hydrogen; or an organic radical; C₁-C₃alkyl; or a polyoxyethylene radical which contains from 1 to 10 ethylene oxide units and the terminal OH group of which may be etherified by a C₁-C₃alcohol.

In relation to the compounds of formula (28), when R₃₇, R₃₈ and R₃₉ is an alkali metal it is preferably potassium or, especially sodium; when R₃₇, R₃₈ and R₃₉ is a group N(R₄₀)₄ in which R₃₀ has its previous significance, it is preferably a mono-, di- or tri-C₁-C₄alkylammonium salt, a mono-, di- or tri-C₂-C₄alkanolammonium salt or a C₁-C₃alkyl ester thereof; when R₄₀ is a C₁-C₃alkyl group, it is preferably a C₁-C₂alkyl group, more preferably a methyl group; and when R₃₀ is polyoxyethylene group, this preferably contains from 2-6 ethylene oxide units.

One preferred class of triazole micronised organic UV absorbers is that having the formula 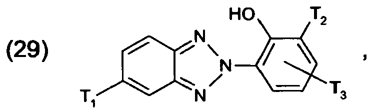 wherein
T₁ is C₁-C₃alkyl or, preferably, hydrogen; or a radical of formula 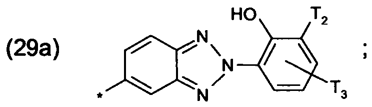 and
T₂ and T₃, independently from each other are C₁-C₁₂alkyl, preferably i-octyl; or C₁-C₄alkyl substituted by phenyl, preferably α,α-dimethylbenzyl.

A further preferred class of triazole micronised organic UV absorbers corresponds to the formula 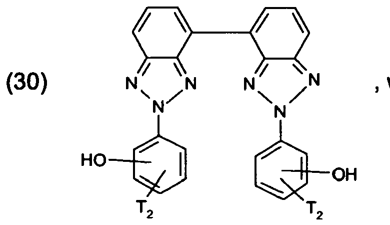 wherein
T₂ has its previous significance.

A still further preferred class of triazole micronised organic UV absorbers corresponds to the formula 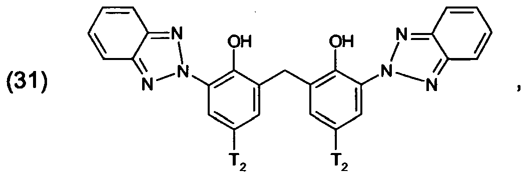 wherein
T₂ is hydrogen; C₁-C₁₂alkyl, preferably iso-octyl, or C₁-C₄alkyl substituted by phenyl, preferably α,α-dimethylbenzyl.

A preferred class of vinyl group-containing amide micronised organic UV absorbers corresponds to the formula:
(32) R₄₁-(Y)ₘ-CO-C(R₄₂)-C(R₄₃)-N(R₄₄)(R₄₅), wherein
R₄₁ is C₁-C₃alkyl, preferably C₁-C₂alkyl, or phenyl optionally substituted by one, two or three substituents selected from OH, C₁-C₃alkyl, C₁-C₃alkoxy or CO-OR₄₆,
R₄₆ C₁-C₃alkyl;
R₄₂, R₄₃, R₄₄ and R₄₅ are the same or different and each is C₁-C₃alkyl, preferably C₁-C₂alkyl; or hydrogen;
Y is -NH- ; or -O-; and
m is 0; or 1.

Preferred compounds of formula (32) are 4-methyl-3-penten-2-one, ethyl-3-methylamino-2-butenoate, 3-methylamino-1-phenyl-2-buten-1-one and 3-methylamino-1-phenyl-2-buten-1-one.

A preferred class of cinnamic acid amide micronised organic UV absorbers corresponds to the formula: 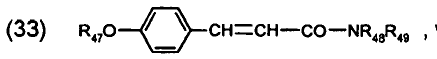 wherein
R₄₇ is hydroxy or C₁-C₄alkoxy, preferably methoxy or ethoxy;
R₄₈ is hydrogen or C₁-C₄alkyl, preferably methyl or ethyl; and
R₄₉ is -(CONH)ₘ-phenyl in which m is 0 or 1 and the phenyl group is optionally substituted by one, two or three substituents selected from OH, C₁-C₃alkyl, C₁-C₃alkoxy or CO-OR_{50;} and
R₅₀ is C₁-C₄alkyl.

Further preferred classes of micronised or micronisable UV absorbers used for the present invention are:
- p-aminobenzoic acid derivatives, typically 2-ethylhexyl-4-dimethylaminobenzoate
- salicylic acid derivatives, typically 2-ethylhexyl salicylate; homosalates; and isopropyl sylicylates;
- benzophenone derivatives, typically 2-hydroxy-4-methoxybenzophenone;
- dibenzoylmethane derivatives, typically 1-(4-tert-butylphenyl)-3-(4-methoxyphenyl)-propane-1,3-dione;
- diphenylacrylates, typically 2-ethylhexyl-2-cyano-3,3-diphenylacrylate and 3-(benzofuranyl)-2-cyanoacrylate;
- 3-imidazol-4-yl-acrylic acid and 3-imidazol-4-yl-acrylate;
- benzofurane derivatives, preferably 2-(p-aminophenyl)benzofuran derivatives, disclosed in EP-A-582 189, US-A-5 338 539, US-A-5 518 713 and EP-A-613 893;
- polymeric UV absorbers, such as the benzylidenemalonate derivatives described, inter alia in EPA-709 080;
- cinnamic acid derivatives, typically the 2-ethylhexyl-4-methoxycinnamate or isoamylate or cinnamic acid derivatives disclosed, inter alia, in US-A-5 601 811 and WO 97/00851;
- camphor derivatives, typically 3-(4'-methyl)benzylidenebornan-2-one, 3-benzylidenebornan-2-one, N-[2(and 4)-2-oxybom-3-ylidenemethyl)benzyl]acrylamide polymer, 3-(4'-trimethylammonium)benzylidenebornan-2-one methylsulfate, 3,3'-(1,4-phenylenedimethine)-bis(7,7-dimethyl-2-oxobicyclo-[2.2.1]heptane-1-methanesulfonic acid) and the salts thereof, 3-(4'-sulfo)benzylidenebornan-2-one and the salts thereof;
- 2-phenylbenzimidazole-5-sulfonic acids and the salts thereof; and
- menthyl-o-aminobenzoate.

Fatty alcohols are generally understood as saturated or unsaturated primary alcohols (1-alkanols) having 6 to 22 carbon atoms. They are obtained by reduction of triglycerides, fatty acids or fatty acid methyl esters.

Preferred combinations of insoluble micronized organic UV-screening actives (component (c₁) and primary C₆-C₂₈ fatty alcohols (component (c₂)) are:

| insoluble micronized organic UV-screening active (c₁) | primary C₆-C₂₈ fatty alcohol (=c₂) |
|---|---|
| Compound of formula (31), wherein T₂ is isooctyl | mixture of linear octanol and linear decanol |
| Compound of formula (4) | mixture of linear octanol and linear decanol |

The micronised organic UV absorber, component (c₁), is preferably produced by the method described in GB-A-2303549, namely by a process which comprises grinding the corresponding organic UV absorber, in coarse particle form, in a grinding apparatus, in the presence of 1 to 50%, preferably 5 to 40% by weight, based on the micronised organic UV absorber, of an alkyl polyglucoside having the formula CₙH₂ₙ₊₁O(C₆H₁₀O₅)ₓH, in which n is an integer ranging from 8 to 16 and x is the mean polymerisation level of the glucoside moiety (C₆H₁₀O₅) and ranges from 1.4 to 1.6, or an ester thereof.

Any known process suitable for the preparation of microparticles can be used for the preparation of the micronised UV absorbers, for example wet-milling, wet-kneading, spray-drying from a suitable solvent, by the expansion according to the RESS process (Rapid Expansion of Supercritical Solutions), by reprecipitation from suitable solvents, including supercritical fluids (GASR process = Gas Anti-Solvent Recrystallisation / PCA process = Precipitation with Compressed Anti-solvents).

The micronised UV absorbers so obtained usually have an average particle size from 0.02 to 2, preferably from 0.03 to 1.5, and more especially from 0.05 to 1.0 micrometer.

The UV absorbers according to component (c₁) can also be used as dry substrates in powder form

The topically applicable water-in-oil (w/o) or water-in-silicone (w/Si) formulation according to the present invention may additionally contain one or more than one further non-micronized UV filter as listed in Tables 1 and 2.

The UV absorbers as described in Tables 1 and 2 below may be added to the topically applicable water-in-oil (w/o) or water-in-silicone (w/Si) formulations according to the present invention in a amounts from 0.1 to 20 % b.w. Mixtures of these UV absorbers can be used, *inter alia,* to improve the solubility or to increase UV absorption.

| Table 1. Suitable non-micronized UV filter substances which can be additionally used with the UV absorbers according to the present invention |
|---|
| p-aminobenzoic acid derivatives, for example 4-dimethylaminobenzoic acid 2-ethylhexyl ester; |
| salicylic acid derivatives, for example salicylic acid 2-ethylhexyl ester; |
| benzophenone derivatives, for example 2-hydroxy-4-methoxybenzophenone and its 5-sulfonic acid derivative; |
| dibenzoylmethane derivatives, for example 1-(4-tert-butylphenyl)-3-(4-methoxyphenyl)-propane-1,3-dione; |
| diphenylacrylates, for example 2-ethylhexyl 2-cyano-3,3-diphenylacrylate, and 3-(benzofuranyl) 2-cyanoacrylate; |
| 3-imidazol-4-ylacrylic acid and esters; |
| benzofuran derivatives, especially 2-(p-aminophenyl)benzofuran derivatives, described in EP-A-582 189, US-A-5 338 539, US-A-5 518 713 and EP-A-613 893; |
| polymeric UV absorbers, for example the benzylidene malonate derivatives described in EP-A-709 080; |
| cinnamic acid derivatives, for example the 4-methoxycinnamic acid 2-ethylhexyl ester and isoamyl ester or cinnamic acid derivatives described in US-A-5 601 811 and WO 97/00851; |
| camphor derivatives, for example 3-(4'-methyl)benzylidene-bornan-2-one, 3-benzylidene-bornan-2-one, N-[2(and 4)-2-oxyborn-3-ylidene-methyl)-benzyl]acrylamide polymer, 3-(4'-trimethylammonium)-benzylidene-boman-2-one methyl sulfate, 3,3'-(1,4-phenylenedimethine)-bis(7,7-dimethyl-2-oxo-bicyclo[2.2.1]heptane-1-methanesulfonic acid) and salts, 3-(4'-sulfo)benzylidene-boman-2-one and salts; camphorbenzalkonium methosulfate; |
| hydroxyphenyltriazine compounds, for example 2-(4'-methoxyphenyl)-4,6-bis(2'-hydroxy-4'-n-octyloxyphenyl)-1,3,5-triazine; 2,4-bis{[4-(3-(2-propyloxy)-2-hydroxy-propyloxy)-2-hydroxy]-phenyl}-6-(4-methoxyphenyl)-1,3,5-triazine; 2,4-bis{[4-(2-ethyl-hexyloxy)-2-hydroxy]-phenyl}-6-[4-(2-methoxyethyl-carboxyl)-phenylamino]-1,3,5-triazine; 2,4-bis{[4-(tris(trimethylsilyloxy-silylpropyloxy)-2-hydroxy]-phenyl}-6-(4-methoxyphenyl)-1,3,5-triazine; 2,4-bis{[4-(2"-methylpropenyloxy)-2-hydroxy]-phenyl}-6-(4-methoxyphenyl)-1,3,5-triazine; 2,4-bis{[4-(1',1',1',3',5',5',5'-heptamethyltrisilyl-2"-methyl-propyloxy)-2-hydroxy]-phenyl}-6-(4-methoxyphenyl)-1,3,5-triazine; 2,4-bis{[4-(3-(2-propyloxy)-2-hydroxy-propyloxy)-2-hydroxy]-phenyl}-6-[4-ethylcarboxy)-phenylamino]-1,3,5-triazine; |
| benzotriazole compounds, for example 2,2'-methylene-bis(6-(2H-benzotriazol-2-yl)-4-(1,1,3,3-tetramethylbutyl)-phenol; |
| trianilino-s-triazine derivatives, for example 2,4,6-trianiline-(p-carbo-2'-ethyl-1'-oxy)-1,3,5-triazine and the UV absorbers disclosed in US-A-5 332 568, EP-A-517 104, EP-A-507 691, WO 93/17002 and EP-A-570 838; |
| 2-phenylbenzimidazole-5-sulfonic acid and salts thereof; |
| menthyl o-aminobenzoates; |
| physical sunscreens coated or not as titanium dioxide, zinc oxide, iron oxides, mica, MnO, Fe₂O₃ Ce₂O₃, Al₂O₃, ZrO₂. (surface coatings: polymethylmethacrylate, methicone (methylhydrogenpolysiloxane as described in CAS 9004-73-3), dimethicone, isopropyl titanium triisostearate (as described in CAS 61417-49-0), metal soaps as magnesium stearate (as described in CAS 4086-70-8), perfluoroalcohol phosphate as C9-15 fluoroalcohol phosphate (as described in CAS 74499-44-8; JP 5-86984 , JP 4-330007)). The primary particle size is an average of 15nm-35nm and the particle size in dispersion is in the range of 100nm - 300nm. |
| aminohydroxy-benzophenone derivatives disclosed in DE 10011317, EP 1133980 and EP 1046391 |
| phenyl-benzimidazole derivatives as disclosed in EP 1167358 |
| the UV absorbers described in "Sunscreens", Eds. N.J. Lowe, N.A.Shaath, Marcel Dekker, Inc. , New York and Basle or in Cosmetics & Toiletries (107), 50ff (1992) also can be used as additional UV protective substances. |

| Table 2: Suitable specific non-micronized UV filter substances and adjuvants which can be additionally used with the UV absorbers according to the present invention | | |
|---|---|---|
| No. | Chemical Name | CAS No. |
| 1 | (+/-)-1,7,7-trimethyl-3-[(4-methylphenyl)methylene]bicyclo-[2.2.1]heptan-2-one; p-methyl benzylidene camphor | 36861-47-9 |
| 2 | 1,7,7-trimethyl-3-(phenylmethylene)bicyclo[2.2.1]heptan-2-one; benzylidene camphor | 15087-24-8 |
| 3 | (2-Hydroxy-4-methoxyphenyl)(4-methylphenyl)methanone | 1641-17-4 |
| 4 | 2,4-dihydroxybenzophenone | 131-56-6 |
| 5 | 2,2',4,4'-tetrahydroxybenzophenone | 131-55-5 |
| 6 | 2-Hydroxy-4-methoxy benzophenone; | 131-57-7 |
| 7 | 2-Hydroxy-4-methoxy benzophenone-5-sulfonic acid | 4065-45-6 |
| 8 | 2,2'-dihydroxy-4,4'-dimethoxybenzophenone | 131-54-4 |
| 9 | 2,2'-Dihydroxy-4-methoxybenzophenone | 131-53-3 |
| 10 | Alpha-(2-oxobom-3-ylidene)toluene-4-sulphonic acid and its salts; Mexoryl SL | 56039-58-8 |
| 11 | 1-[4-(1,1-dimethylethyl)phenyl]-3-(4-methoxyphenyl)propane-1,3-dione; avobenzone | 70356-09-1 |
| 12 | Methyl N,N,N-trimethyl-4-[(4,7,7-trimethyl-3-oxobicyclo[2,2,1]hept-2-ylidene)methyl]anilinium sulphate; Mexoryl SO | 52793-97-2 |
| 22 | 3,3,5-Trimethyl cyclohexyl-2-hydroxy benzoate; homosalate | 118-56-9 |
| 23 | Isopentyl p-methoxycinnamate; isoamyl methoxy cinnamate | 71617-10-2 |
| 27 | Menthyl-o-aminobenzoate | 134-09-8 |
| 28 | Menthyl salicylate | 89-46-3 |
| 29 | 2-Ethylhexyl 2-cyano,3,3-diphenylacrylate; octocrylene | 6197-30-4 |
| 30 | 2- ethylhexyl 4- (dimethylamino)benzoate | 21245-02-3 |
| 31 | 2- ethylhexyl 4- methoxycinnamate; octyl methoxy cinnamate | 5466-77-3 |
| 32 | 2- ethylhexyl salicylate | 118-60-5 |
| 33 | Benzoic acid, 4, 4', 4"- (1, 3, 5- triazine- 2, 4, 6- triyltriimino)tris-, tris(2-ethylhexyl)ester; 2,4,6-Trianilino-(p-carbo-2'-ethylhexyl-1'-oxi)-1,3,5-triazine; octyl triazone | 88122-99-0 |
| 34 | 4- aminobenzoic acid | 150-13-0 |
| 35 | Benzoic acid, 4-amino-, ethyl ester, polymer with oxirane | 113010-52-9 |
| 38 | 2- phenyl- 1H- benzimidazole- 5- sulphonic acid; phenylbenzimidazolsulfonic acid | 27503-81-7 |
| 39 | 2-Propenamide, N-[[4-[(4,7,7-trimethyl-3-oxobicyclo[2.2.1]hept-2-ylidene)methyl]phenyl]methyl]-, homopolymer | 147897-12-9 |
| 40 | Triethanolamine salicylate | 2174-16-5 |
| 41 | 3, 3'-(1,4-phenylenedimethylene)bis[7, 7-dimethyl- 2-oxo-bicyclo[2.2.1]heptane-1 methanesulfonic acid]; Cibafast H | 90457-82-2 |
| 42 | Titanium dioxide | 13463-67-7 |
| 44 | Zinc oxide | 1314-13-2 |
| 45 | 2,4-bis{[4-(2-ethylhexyloxy)-2-hydroxy]-phenyl}-6-(4-methoxyphenyl)-(1,3,5)-triazine; Tinosorb S | 187393-00-6 |
| 46 | 1H-Benzimidazole-4,6-disulfonic acid, 2,2'-(1,4-phenylene)bis-, disodium salt | 180898-37-7 |
| 47 | Benzoic acid, 4,4'-[[6-[[4-[[(1,1-dimethylethyl)amino]carbonyl]phenyl]amino]1,3,5-triazine-2,4-diyl]diimino]bis-, bis(2-ethylhexyl)ester; diethylhexyl butamido triazone; Uvasorb HEB | 154702-15-5 |
| 48 | Phenol, 2-(2H-benzotriazol-2-yl)-4-methyl-6-[2-methyl-3-[1,3,3,3-tetramethyl-1-[(trimethylsilyl)oxy]disiloxanyl]propyl]-; drometrizole trisiloxane; Mexoryl XL | 155633-54-8 |
| 49 | Dimethicodiethylbenzalmalonate; Polysilicone 15; Parsol SLX | 207574-74-1 |
| 50 | Benzenesulfonic acid, 3-(2H-benzotriazol-2-yl)-4-hydroxy-5-(1-methylpropyl)-, monosodium salt ; Tinogard HS | 92484-48-5 |
| 51 | Benzoic acid, 2-[4-(diethylamino)-2-hydroxybenzoyl]-, hexyl ester; Uvinul a plus | 302776-68-7 |
| 52 | 1-Dodecanaminium, N-[3-[[4-(dimethylamino)benzoyl]amino]propyl]-N,N-dimethyl-, salt with 4-methylbenzenesulfonic acid (1:1); Escalol HP610 | 156679-41-3 |
| 53 | 1-Propanaminium, N,N,N-trimethyl-3-[(1-oxo-3-phenyl-2-propenyl)-amino]-, chloride | 177190-98-6 |
| 54 | 1H-Benzimidazole-4,6-disulfonic acid, 2,2'-(1,4-phenylene)bis- | 170864-82-1 |
| 55 | 1,3,5-Triazine, 2,4,6-tris(4-methoxyphenyl)- | 7753-12-0 |
| 56 | 1,3,5-Triazine, 2,4,6-tris[4-[(2-ethylhexyl)oxy]phenyl]- | 208114-14-1 |
| 57 | 1-Propanaminium, 3-[[3-[3-(2H-benzotriazol-2-yl)-5-(1,1-dimethylethyl)-4-hydroxyphenyl]-1-oxopropyl]amino]-N,N-diethyl-N-methyl-, methyl sulfate (salt) | 340964-15-0 |
| 58 | 2-Propenoic acid, 3-(1H-imidazol-4-yl)- | 104-98-3 |
| 59 | Benzoic acid, 2-hydroxy-, [4-(1-methylethyl)phenyl]methyl ester | 94134-93-7 |
| 60 | 1,2,3-Propanetriol, 1-(4-aminobenzoate); glyceryl PABA | 136-44-7 |
| 61 | Benzeneacetic acid, 3,4-dimethoxy-α-oxo- | 4732-70-1 |
| 62 | 2-Propenoic acid, 2-cyano-3,3-diphenyl-, ethyl ester | 5232-99-5 |
| 63 | Anthralinic acid, p-menth-3-yl ester | 134-09-8 |
| 64 | 2,2'-bis(1,4-phenylene)-1H-benzimidazole-4,6-disulphonic acid mo-no sodium salt or Disodium phenyl dibenzimidazole tetrasulfonate or Neoheliopan AP | 349580-12-7, |
| 65 | 1,3,5-Triazine-2,4,6-triamine, N,N'-bis[4-[5-(1,1-dimethylpropyl)-2-benzoxazolyl]phenyl]-N"-(2-ethylhexyl)- or Uvasorb K2A | 288254-16-0 |
| 66 | Merocyanine derivatives as described in WO 2004006878 and in IPCOM000022279D | |
| 67 | 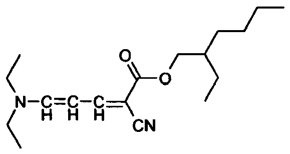 | |
| 68 | sterols (cholesterol, lanosterol, phytosterols), as described in WO0341675 | |
| 69 | mycosporines and/or mycosporine-like amino acids as described in WO2002039974, e.g. Helioguard 365 from Milbelle AG, isolated mycosporine like amino acids from the red alga porphyra umbilicalis (INCI: Porphyra Umbilicalis) that are encapsulated into liposomes,) | |
| 70 | alpha-lipoic-acid as described in DE 10229995 | |
| 71 | synthetic organic polymers as described in EP 1371358, [0033]-[0041] | |
| 72 | phyllosilicates as described in EP 1371357 [0034]-[0037] | |
| 73 | silica compounds as described in EP1371356, [0033]-[0041] | |
| 74 | inorganic particles as described in DE10138496 [0043]-[0055] | |
| 75 | latex particles as described in DE10138496 [0027]-[0040] | |
| 76 | 1H-Benzimidazole-4,6-disulfonic acid, 2,2'-(1,4-phenylene)bis-, disodium salt ; Bisimidazylate; Neo Heliopan APC | 180898-37-7 |

The topically applicable formulations of the present invention comprise at least a water phase and an oil-phase.

They are preferably formulated as water-in-oil (w/o) or water-in-silicone (w/Si) emulsions or microemulsions.

They may contain low molecular weight emulsifiers selected from non-ionic, cationic, amphoteric and anionic emulsifiers from 0.1 to 20 % bw..

The UV-photoprotecting composition (c) is prepared by incorporating component (c₂) into the UV absorber dispersion which contains the micronized UV absorber by simply mixing the 2 components.

The so obtained UV-photoprotecting composition (c) is preferably incorporated into the oil phase of the W/O emulsion.

The simple adding of such UV-photoprotecting composition helps to stabilize or even eliminate the crystal growth of the selected micronized organic UV-screening agent within the oil phase of the W/O or W/Si emulsion, or int the Si-phase of the w/Si emulsion respectively.

The final W/O or W/Si formulations according to the present invention may exist in a wide variety of presentation forms, for example:
- in the form of liquid preparations as a W/O, O/W, O/W/O, W/O/W or PIT emulsion and all kinds of microemulsions,
- in the form of a gel,
- in the form of an oil, a cream, milk or lotion,
- in the form of a stick,
- in the form of a spray (spray with propellent gas or pump-action spray) or an aerosol,
- in the form of a foam, or
- in the form of a paste.

Of special importance as cosmetic preparations for the skin are light-protective preparations, such as sun milks, lotions, creams, oils, sunblocks or tropicals, pretanning preparations or after-sun preparations, also skin-tanning preparations, for example self-tanning creams. Of particular interest are sun protection creams, sun protection lotions, sun protection milk and sun protection preparations in the form of a spray.

Of special importance as cosmetic preparations for the hair are the above-mentioned preparations for hair treatment, especially hair-washing preparations in the form of shampoos, hair conditioners, hair-care preparations, e.g. pretreatment preparations, hair tonics, styling creams, styling gels, pomades, hair rinses, treatment packs, intensive hair treatments, hair-straightening preparations, liquid hair-setting preparations, hair foams and hairsprays. Of special interest are hair-washing preparations in the form of shampoos.

As water- and oil-containing emulsions or microemulsions the preparations contain, for example,
from 0.1 to 30 % by weight, preferably from 0.1 to 15 % by weight and especially from 0.5 to 10 % by weight, based on the total weight of the composition, of an effective UV-photoprotecting composition (= component (c));
from 1 to 60 % by weight, especially from 5 to 50 % by weight and preferably from 10 to 35 % by weight, based on the total weight of the composition, of at least one oil component,
from 0 to 30 % by weight, especially from 1 to 30 % by weight und preferably from 4 to 20 % by weight, based on the total weight of the composition, of at least one emulsifier,
from 10 to 90 % by weight, especially from 30 to 90 % by weight, based on the total weight of the composition, of water, and
from 0 to 88.9 % by weight, especially from 1 to 50 % by weight, of further cosmetically acceptable adjuvants.

The topically applicable water-in-oil (w/o) or water-in-silicone (w/si) formulation may also contain one or one more additional compounds as esters of fatty acids,natural or synthetic triglycerides including glyceryl esters and derivatives, pearlescent waxes, hydrocarbon oils, silicones or siloxanes (organosubstituted polysiloxanes), fluorinated or perfluorinated oils, super-fatting agents, surfactants, consistency regulators/thickeners and rheology modifiers, polymers, biogenic active ingredients, deodorising active ingredients, anti-dandruff agents, antioxidants, hydrotropic agents, preservatives and bacteria-inhibiting agents, perfume oils, colourants, insect repellents or polymeric beads or hollow spheres as spf enhancers.

Cosmetic or pharmaceutical formulations are contained in a wide variety of cosmetic preparations like skin-care preparations, bath preparations, cosmetic personal care preparations, foot-care preparations, light-protective preparations, skin-tanning preparations, depigmenting preparations, insect-repellents, deodorants preparations for cleansing and caring for blemished skin, e.g. synthetic detergents (solid or liquid), peeling or scrub preparations or peeling masks; hair-removal preparations in chemical form (depilation), - shaving preparations, fragrance preparations, cosmetic hair-treatment preparations,

The cosmetic preparation according to the invention is distinguished by excellent protection of human skin against the damaging effect of sunlight.

### Examples

### Example 1: W/O SunscreenContaining TINOSORB^{®}M & Tinosorb^{®}OMC

(c): reference example

| Composition | | Ex. 1a⁽²⁾ | Ex. 1b |
|---|---|---|---|
| | INCl-Name | %w/w (as supplied | % w/w (as supplied) |
| Part A | Glyceryl Oleate | 4.50 | 4.50 |
| | PEG-7 Hydrogenated Castor Oil | 1.50 | 1.50 |
| | Hydrogenated Castor Oil | 0.50 | 0.50 |
| | Microcrystalline Wax | 2.00 | 2.00 |
| | Ethylhexyl Methoxycinnamate | 5,00 | 5,00 |
| | Beeswax | 1.50 | 1.50 |
| | C12-15 Alkyl Benzoate | 8.00 | 8.00 |
| | Isopropyl Isostearate | 5.00 | 5.00 |
| | Mineral Oil | 5.00 | 5.00 |
| Part B | Aqua | qs to 100 | qs to 100 |
| | Magnesium Sulfate | 0.50 | 0.50 |
| | Citric Acid | 0.05 | 0.05 |
| Part C | Methylene Bis-Benzotriazolyl Tetramethylbutyl-phenol (and) Aqua (and) Propylene Glycol (and) Decyl Glucoside (and) Xanthan Gum | 3,75 a.i. | 3,75 a.i. |
| | Butyloctanol | 1.40 | - |
| | Blend of Octanol and Decanol | - | 0.90 |

Manufacturing instruction: Heat separately part A to 80°C and B to 80°C. Slowly add part B to part A under high stirring. Decrease the speed till moderate stirring 500 rpm. Let cool down to 35°C and add part C.

### Example 2:W/O-Sunscreen (c): reference example

| Composition | | Ex. 2a⁽²⁾ | Ex. 2b |
|---|---|---|---|
| | INCl-Name | %w/w (as supplied) | % w/w (as supplied) |
| Part A | PEG-30 Dipolyhydroxystearate | 2.00 | 2.00 |
| | Cetearyl Isononanoate | 5.00 | 5.00 |
| | Hydrogenated Polydecene | 3.50 | 3.50 |
| | Ethylhexyl Methoxycinnamate | 5.00 | 5.00 |
| | C12-15 Alkyl Benzoate | 4.00 | 4.00 |
| | Cyclomethicone | 3.00 | 3.00 |
| Part B | Aqua | qs to 100 | qs to 100 |
| | Sorbeth-30 | 3.00 | 3.00 |
| | Magnesium Sulfate | 0.70 | 0.70 |
| Part C | Propylene Glycol (and) Diazolidinyl Urea (and) Methylparaben (and) Propylparaben | 0.30 | 0.30 |
| | Methylene Bis-Benzotriazolyl Tetramethyl-butylphenol (and) Aqua (and) Propylene Glycol (and) Decyl Glucoside (and) Xanthan Gum | 3,75 a.i. | 3,75 a.i. |
| | Butyloctanol | 2.80 | - |
| | Blend of Octanol and Decanol | - | 1.40 |

Manufacturing instruction:Heat part A and part B separately to 80C under stirring. Add part B to part A with continuous stirring. Homogenize for 30 sec with Ultra Turrax at 11000 rpm. Let cool down to 40°C and add part C. Cool down to room temperature under continuous stirring.

### Example 3: W/O Sunscreen

| Composition | | Ex. 3a^{(c)} | Ex. 3b |
|---|---|---|---|
| | INCl-Name | % w/w (as supplied) | % w/w (as supplied) |
| Part A | PEG-30 Dipolyhydroxystearate | | 5.00 |
| | Cetearyl lsononanoate | | 2.50 |
| | Hydrogenated Polydecene | | 6,50 |
| | Ethylhexyl Methoxycinnamate | | 4,00 |
| | C12-15 Alkyl Benzoate | | 0.50 |
| Part B | Aqua | | Qs to 100 |
| | Sorbeth-30 | | 1.00 |
| Part C | Propylene Glycol (and) Diazolidinyl Urea (and) Methylparaben (and) Propylparaben | | 3,75 a.i. |
| | Methylene Bis-Benzotriazolyl Tetramethylbutylphenol (and) Aqua (and) Propylene Glycol (and) Decyl Glucoside (and) Xanthan Gum | | 0.30 |
| | Butyloctanol | 1.15 | - |
| | Blend of Octanol and Decanol | - | 1.15 |

| | | | |
|---|---|---|---|
| (c): reference example Appearance: white viscous emulsion Viscosity (Brookfield DVIII+LV/T-C/50rpm): 80000-100000 mPas | | | |

Manufacturing instruction: Heat part A to70°C under continuous stirring. Add part B to part A and homogenize for a minute by 11000 rpm. Let cool down to room temperature and add part C under gentle stirring

### Example 4: W/O Sun Emulsion

| Composition | | Ex. 4a^{(c)} | Ex. 4b |
|---|---|---|---|
| | INCl-Name | % w/w (as supplied) | % w/w (as supplied) |
| Part A | Sorbitan Isostearate | 5.00 | 5.00 |
| | Lanolin Alcohol | 1.00 | 1.00 |
| | PEG-7 Hydrogenated Castor Oil | 1.50 | 1.50 |
| | Microcrystalline Wax | 1.50 | 1.50 |
| | Vaseline | 3.00 | 3.00 |
| | Caprylic/Capric Triglyceride | 1,50 | 1,50 |
| | Ethylhexyl Methoxycinnamate | 5.00 | 5.00 |
| | Paraffinium Subliquidum | 5.00 | 5.00 |
| Part B | Aqua | qs to 100 | qs to 100 |
| | Magnesium Sulfate | 0.70 | 0.70 |
| Part C | Methylene Bis-Benzotriazolyl Tetramethyl-butylphenol (and) Aqua (and) Propylene Glycol (and) Decyl Glucoside (and) Xanthan Gum | 3,75 a.i. | 3,75 a.i. |
| | Propylene Glycol (and) Diazolidinyl Urea (and) Methylparaben (and) Propylparaben | 0.30 | 0.30 |
| | Butyloctanol | 0.80 | |
| | Blend of Octanol and Decanol | | 0.80 |

| | | | |
|---|---|---|---|
| * measurement made on Labsphere UV Transmittance Analyzer 1.2µl/cm² on PMMA (c) :reference example | | | |

Manufacturing instruction: Heat phase A to 75°C. Add part B to part A and homogenize for 30 sec. at 11000rpm. Let cool down to 50°C and add part C. Continuous stirring until room temperature.

## Claims

1. A topically applicable water-in-oil (w/o) or water-in-silicone (w/Si) formulation comprising:
(a) 10 to 90 % b.w. of at least one aqueous phase:
(b) 1 to 60 % b.w. of at least one fatty phase;
(c) 0.1 to 30 % b.w. of an effective UV-photoprotecting composition comprising
(C₁) 20% to 99% of at least one insoluble micronized organic UV-screening active with a mean particle size ranging from 0,01 µm to 2 µm;
(C₂) 0.5% to 80% of a mixture of linear octanol and linear decanol;
0 to 30 % b.w. of at least one emulsifier: and
0 to 88.9 % b.w. of further cosmetically acceptable adjuvants_{.}

2. Formulation according to claim 1, wherein
(C₁) is selected from a triazine, a benzotriazole, a benzophenone, a vinyl group-containing amide, a cinnamic acid amide and a sulfonated benzimidazole UV absorber.

3. Formulation according to claim 1 or 2, wherein the triazine UV absorber corresponds to the formula 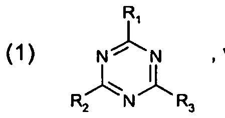 wherein
R₁, R₂ and R₃, independently from each other, are hydrogen; hydroxy; C₁-C₃alkoxy; NH₂; NHR₄; N(R₄)₂; OR₄; C₆-C₁₂aryl; phenoxy; anilino; pyrrolo; in which the respective phenyl, phenoxy, anilino or pyrrolo moieties are not substituted or substituted by one, two or three substitutents selected from OH, carboxy, CO-NH₂, C₁-C₁₂alkyl, C₁-C₁₂alkoxy, a methylidenecamphor group, a group -(CH=CH)ₘC(=O)-OR₄, a group 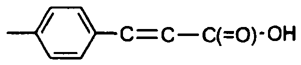 or the corresponding alkali metal, ammonium, mono-, di- or tri-C₁-C₄alkylammonium, mono-, di- or tri-C₂-C₄alkanolammonium salts, or the C₁-C₃alkyl esters thereof or by a radical of formula 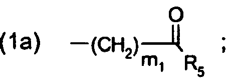 R₄ is C₁-C₅alkyl;
R₅ is hydroxy; C₁-C₅alkyl that is unsubstituted or substituted by one or more OH groups; C₁-C₅alkoxy; amino; mono- or di-C₁-C₅alkylamino; M; a radical of formula 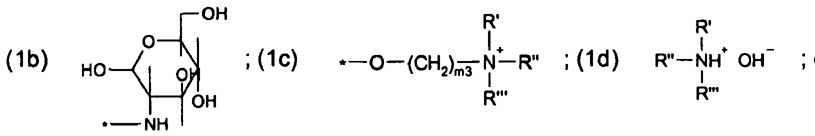 or 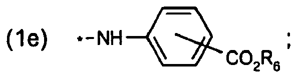 R', R" and R"' independently of the other are C₁-C₁₄alkyl that is unsubstituted or substituted by one or more OH groups;
R₆ is hydrogen; M; C₁-C₅alkyl; or a radical of the formula 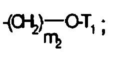 M is a metal cation;
T₁ is hydrogen; or C₁-C₈alkyl;
m is 0 or 1;
m₁ is from 1 to 5;
m₂ is from 1 to 4; and
m₃ is from 2 to 14.

4. Formulation according to claim 1 or 2, wherein the triazine UV absorber corresponds to formula (1), wherein
R₁. R₂ and R₃ independently from each other are a radical of formula 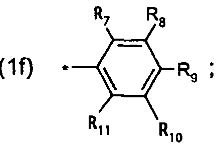 or 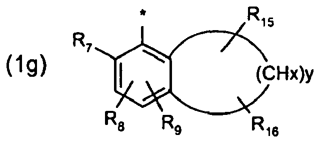 R₇ and R₁₁ independently from each other are hydrogen; C₁-C₁₈alkyl; or C₆-C₁₂aryl;
R₈, R₉ and R₁₀, independently from each other, are hydrogen; or a radical of formula 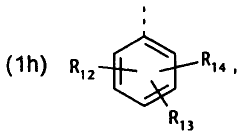 wherein, in formula (1f), at least one of the radicals R₈, R₉ and
R₁₀ are a radical of formula (1 h);
R₁₂, R₁₃, R₁₄, R₁₅ and R₁₆ independently from each other are hydrogen; hydroxy; halogen; C₁-C₁₈alkyl; C₁-C₁₈alkoxy; C₆-C₁₂aryl; biphenylyl; C₆-C₁₂aryloxy; C₁-C₁₈alkylthio; carboxy; -COOM; C₁-C₁₈-alkylcarboxyl; aminocarbonyl; mono- or di-C₁-C₁₈alkylamino; C₁-C₁₀acylamino; or -COOH;
M is an alkali metal ion;
x is 1 or 2; and
y is a number from 2 to 10.

5. Formulation according to claim 4, wherein the triazines correspond to formula 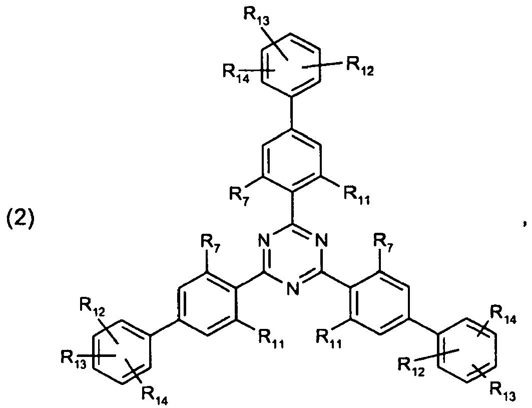 wherein
R₇, R₁₁, R₁₂, R₁₃ und R₁₄ are defined as in claim 4.

6. Formulation according to claim 5, wherein
R₇ and R₁₁ are hydrogen.

7. Formulation according to claim 4, wherin component (c₁) relates to compounds of formula 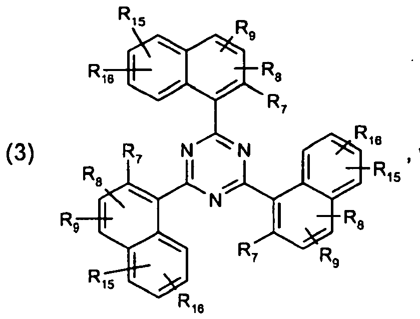 wherein
R₇, R₈, R₉, R₁₅ and R₁₆ are defined as in claim 4.

8. Formulation according to claim 7, wherein
R₇, R₈, R₉, R₁₅ and R₁₆ are hydrogen; or, independently from each other, C₁-C₁₈alkyl.

9. Formulation according to claim 5, wherein component (c₁) corresponds to the formula

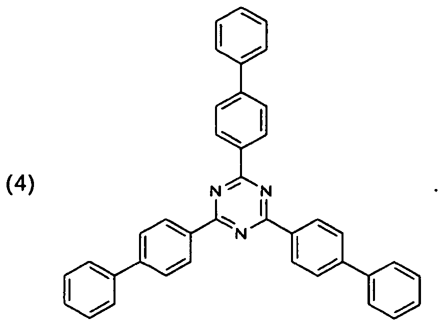

10. Formulation according to any of claims 1 to 3, wherein the triazine UV absorber has the formula 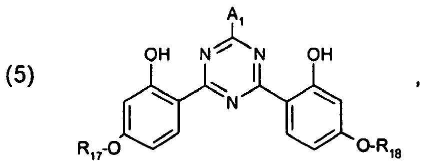 wherein
R₁₇ and R₁₈, independently of one another, are C₁-C₁₈alkyl; C₂-C₁₈alkenyl; a radical of the formula -CH₂-CH(-OH)-CH₂-O-T₁; or a radical of the formula 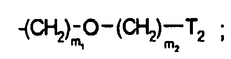 a radical of the formula 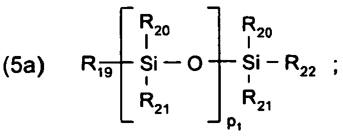 R₁₉ is a direct bond; a straight-chain or branched C₁-C₄alkylene radical or a radical of the formula 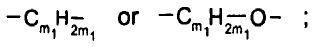 R₂₀, R₂₁ and R₂₂, independently of one another, are C₁-C₁₈alkyl; C₁-C₁₈alkoxy or a radical of the formula 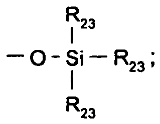 R₂₃ is C₁-C₅alkyl;
T₁ and T₂, independently from each other, are hydrogen; or C₁-C₈alkyl;
m₁, m₂ and m₃, independently of one another, are 1 to 4;
p₁ is 0; or a number from 1 to 5;
A₁ is a radical of the formula 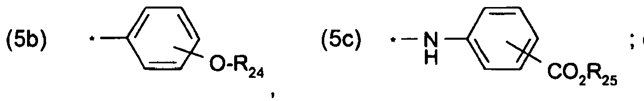 or of the formula 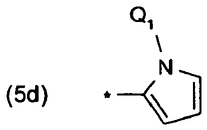 R₂₄ is hydrogen; C₁-C₁₀alkyl, -(CH₂CHR₂₆-O)_{n₁}-R₂₅ ; a -CH₂-CH(-OH)-CH₂-O-T₁ ; or radical of the formula 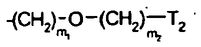 R₂₅ is hydrogen; M; C₁-C₅alkyl; or a radical of the formula -(CH₂)ₘ₂-O-T₁ ;
R₂₆ is hydrogen; or methyl;
Q₁ C₁-C₁₈alkyl;
M is a metal cation; and
n₁ is 1-16.

11. A sun screen formulation according to claim 1 or 2 wherein the triazine compound according to component (c₁) corresponds to formula 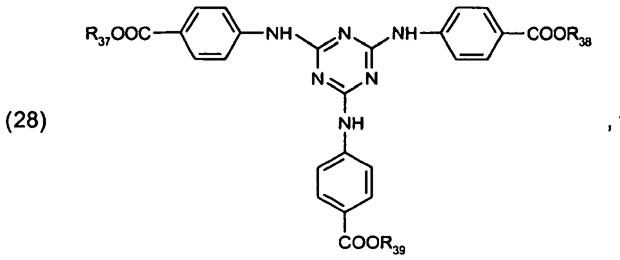 wherein
R₃₇, R₃₈ and R₃₉, independently from each other are hydrogen; an alkali metal; an ammonium group N⁺(R₄₀)₄;
R₄₀ is hydrogen; or an organic radical; C₁-C₃alkyl; or a polyoxyethylene radical which contains from 1 to 10 ethylene oxide units and the terminal OH group of which may be etherified by a C₁-C₃alcohol.

12. Formulation according to claim 1 or 2 in which the triazole organic UV absorber has the formula 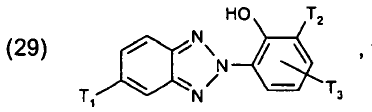 wherein
T₁ is C₁-C₃alkyl or, preferably, hydrogen; or a radical of formula 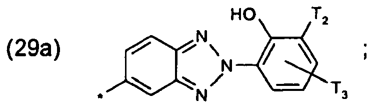 and
T₂ and T₃, independently fro each other are C₁-C₁₂alkyl, preferably i-octyl; or C₁-C₄alkyl substituted by phenyl, preferably α,α-dimethylbenzyl.

13. Formulation according to claim 2 in which the benzotriazole organic UV absorber corresponds to the formula 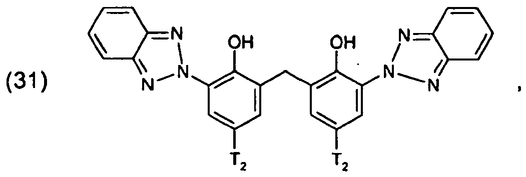 wherein
T₂ is hydrogen; C₁-C₁₂alkyl; or C₁-C₄alkyl substituted by phenyl.

14. Formulation as according to claim 13 wherein
T₂ is iso-octyl.

15. Formulation according to any of the preceding claims wherein the sun screen formulation additionally comprises an oil-soluble organic UV absorber selected from a p-aminobenzoic acid derivative; a salicylic acid derivative; a benzophenone derivative; a dibenzoylmethane derivative; a diphenylacrylate derivative; a benzofuran derivative; a polymeric UV absorber containing one or more silico-organic residues; a cinnamate ester; a camphor derivative; a trianilino-s-triazine derivative; phenylbenzimidazole sulfonic acid or one of its salts; urocanic acid (3-imidazol-4-yl-acrylic acid) or its ethyl ester; menthyl anthranilate; a benzotriazole; a hydroxyphenyltriazine derivative; and a bis-resorcinol-dialkylaminotriazine.

## Patentansprüche

1. Topisch anwendbare Wasser-in-Öl- (w/o) oder Wasser-in-Silicon- (w/Si)-Formulierung, umfassend:
(a) 10 bis 90 Gew. -% wenigstens einer wässrigen Phase;
(b) 1 bis 60 Gew.-% wenigstens einer Fettphase;
(c) 0,1 bis 30 Gew.-% einer wirksamen UV-Lichtschutzzusammensetzung, umfassend
(C₁) 20 % bis 99 % wenigstens eines unlöslichen mikronisierten organischen UV-abschirmenden Wirkstoffs mit einer mittleren Teilchengröße im Bereich von 0,01 µm bis 2 µm;
(C₂) 0,5 % bis 80 % eines Gemischs von linearem Octanol und linearen Decanol;
0 bis 30 Gew.-% wenigstens eines Emulgators; und
0 bis 88,9 Gew. -% weiterer kosmetisch verträglicher Hilfsstoffe.

2. Formulierung gemäß Anspruch 1, wobei (C₁) ausgewählt ist aus einem Triazin-, einem Benzotriazol-, einem Benzophenon-, einem Vinylgruppe-enthaltenden Amid-, einem Zimtsäureamid- und einem sulfonierten Benzimidazol-UV-Absorber.

3. Formulierung gemäß Anspruch 1 oder 2, wobei der Triazin-UV-Absorber der Formel 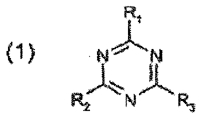 entspricht, wobei
R₁, R₂ und R₃ unabhängig voneinander Wasserstoff; Hydroxy; C₁-C₃-Alkoxy; NH₂; NHR₄; N (R₄) 2; OR₄; C₆-C₁₂-Aryl; Phenoxy; Anilino; Pyrrolo sind; wobei die entsprechenden Phenyl-, Phenoxy-, Anilino- und Pyrrolo-Einheiten nicht substituiert sind oder substituiert mit einem, zwei oder drei Substituenten, ausgewählt aus OH, Carboxy, CO-NH₂, C₁-C₁₂-Alkyl, C₁-C₁₂-Alkoxy, einer Methylidencamphergruppe, einer Gruppe -(CH=CH)ₘC(=O)-OR₄, einer Gruppe 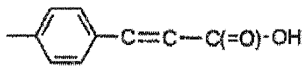 oder den entsprechenden Alkalimetall-, Ammonium-, Mono-, Di-, oder Tri-C₁-C₄-alkylammonium-, Mono-, Di-, oder Tri-C₂-C₄-alkanolammoniumsalzen, oder den C₁-C₃-Alkylestern davon oder mit einem Rest der Formel 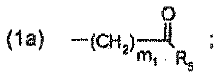 R₄ C₁-C₅-Alkyl ist;
R₅ Hydroxy; C₁-C₅-Alkyl, das nichtsubstituiert ist oder substituiert mit einer oder mehreren OH-Gruppen; C₁-C₅-Alkoxy; Amino; Mono- oder Di-C₁-C₅-alkylamino; M; ein Rest der Formel 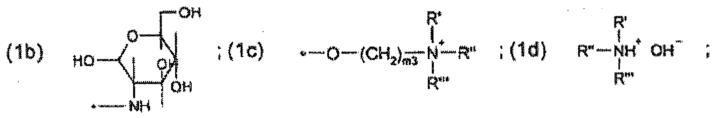 oder 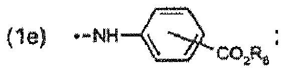 ist;
R', R" und R'" unabhängig voneinander C₁-C₁₄-Alkyl, das nichtsubstituiert ist oder substituiert mit einer oder mehreren OH-Gruppen, sind;
R₆ Wasserstoff; M; C₁-C₅-Alkyl; oder ein Rest der Formel 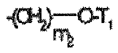 ist;
M ein Metallkation ist;
T₁ Wasserstoff; oder C₁-C₈-Alkyl ist;
m 0 oder 1 ist;
m₁ von 1 bis 5 ist;
m₂ von 1 bis 4 ist; und
m₃ von 2 bis 14 ist.

4. Formulierung gemäß Anspruch 1 oder 2, wobei der Triazin-UV-Absorber der Formel (1) entspricht, wobei
R₁, R₂ und R₃ unabhängig voneinander ein Rest der Formel 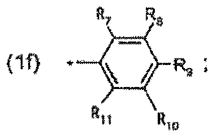 oder 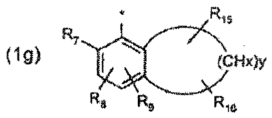 sind;
R₇ und R₁₁ unabhängig voneinander Wasserstoff; C₁-C₁₈-Alkyl; oder C₆-C₁₂-Aryl sind;
R₈, R₉ und R₁₀ unabhängig voneinander Wasserstoff; oder ein Rest der Formel 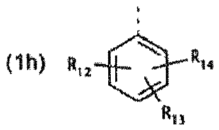 sind, wobei in Formel (1f) wenigstens einer der Reste R₈, R₉ und R₁₀ ein Rest der Formel (1h) ist;
R₁₂, R₁₃, R₁₄, R₁₅ und R₁₆ unabhängig voneinander Wasserstoff; Hydroxy; Halogen; C₁-C₁₈-Alkyl; C₁-C₁₈-Alkoxy; C₆-C₁₂-Aryl; Biphenylyl; C₆-C₁₂-Aryloxy; C₁-C₁₈-Alkylthio; Carboxy; -COOM; C₁-C₁₈-Alkylcarboxy; Aminocarbonyl; Mono- oder Di-C₁-C₁₈-alkylamino; C₁-C₁₀-Acylamino; oder COOH sind;
M ein Alkalimetallion ist;
x 1 oder 2 ist; und
y eine Zahl von 2 bis 10 ist.

5. Formulierung gemäß Anspruch 4, wobei die Triazine der Formel 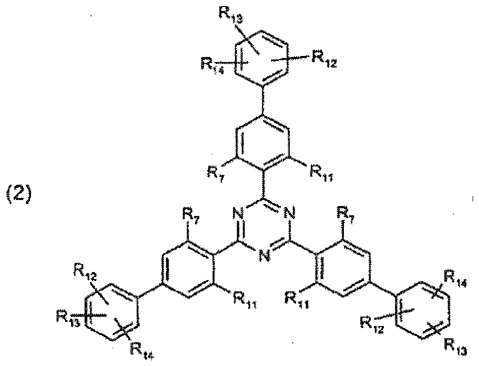 entsprechen, wobei R₇, R₁₁, R₁₂, R₁₃ und R₁₄ wie in Anspruch 4 definiert sind.

6. Formulierung gemäß Anspruch 5, wobei R₇ und R₁₁ Wasserstoff sind.

7. Formulierung gemäß Anspruch 4, wobei die Komponente (C₁) Verbindungen der Formel 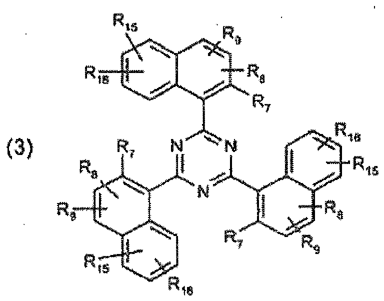 entspricht, wobei R₇, R₈, R₉, R₁₅ und R₁₆ wie in Anspruch 4 definiert sind.

8. Formulierung gemäß Anspruch 7, wobei
R₇, R₈, R₉, R₁₅ und R₁₆ Wasserstoff; oder, unabhängig voneinander, C₁-C₁₈-Alkyl sind.

9. Formulierung gemäß Anspruch 5, wobei die Komponente (C₁) Verbindungen der Formel 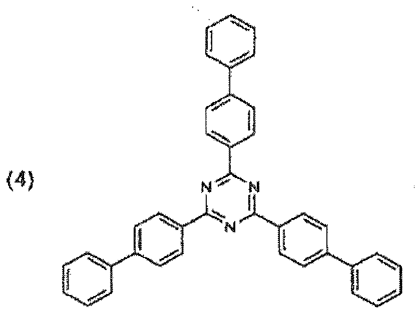 entspricht.

10. Formulierung gemäß einem der Ansprüche 1 bis 3, wobei der Triazin-UV-Absorber die Formel 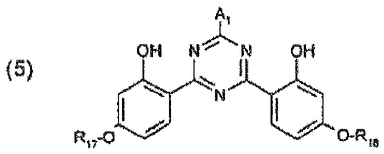 aufweist, wobei
R₁₇ und R₁₈ unabhängig voneinander C₁-C₁₈-Alkyl; C₂-C₁₈-Alkenyl.; ein Rest der Formel -CH₂-CH(-OH)-CH₂-O-T₁; oder ein Rest der Formel 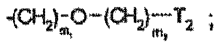 ein Rest der Formel 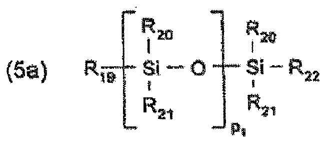 sind;
R₁₉ eine direkte Bindung; ein geradkettiger oder verzweigter C₁-C₄-Alkylenrest oder ein Rest der Formel 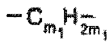 oder 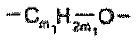 ist;
R₂₀, R₂₁ und R₂₂ unabhängig voneinander C₁-C₁₈-Alkyl; C₁-C₁₈-Alkoxy oder ein Rest der Formel 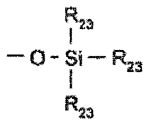 sind;
R₂₃ C₁-C₅-Alkyl ist;
T₁ und T₂ unabhängig voneinander Wasserstoff; oder C₁-C₈-Alkyl sind;
m₁ m₂ und m₃ unabhängig voneinander 1 bis 4 sind;
p₁ 0; oder eine Zahl von 1 bis 5 ist;
A₁ ein Rest der Formel 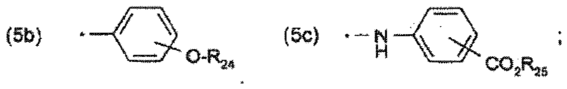
oder der Formel 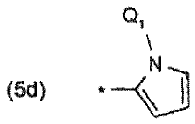 ist;
R₂₄ Wasserstoff; C₁-C₁₀-Alkyl;
**(CH₂CHR₂₆-O)ₙ₁ -R₂₅;**
ein -CH₂-CH(-OH)-CH₂-O-T₁; oder ein Rest der Formel 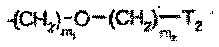 ist;
R₂₅ Wasserstoff; M; C₁-C₅-Alkyl; oder ein Rest der
Formel
-(CH₂)_{m₂}-O-T₁
ist;
R₂₆ Wasserstoff; oder Methyl ist;
Q₁ C₁-C₁₈-Alkyl ist;
M ein Metallkation ist; und
n₁ 1-16 ist.

11. Sonnenschutzformulierung gemäß Anspruch 1 oder 2, wobei die Triazinverbindung gemäß Komponente (C₁) der Formel 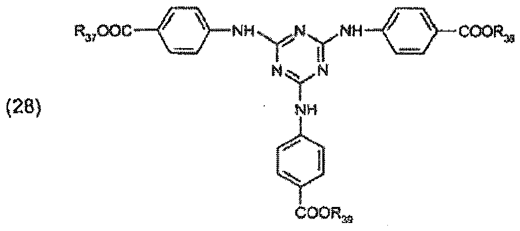 entspricht, wobei
R₃₇, R₃₈ und R₃₉ unabhängig voneinander Wasserstoff; ein Alkalimetall; eine Ammoniumgruppe N⁺(R₄₀)4; sind
R₄₀ Wasserstoff; oder ein organischer Rest; C₁-C₃-Alkyl; oder ein Polyoxyethylenrest, der 1 bis 10 Ethylenoxideinheiten enthält und dessen endständige OH-Gruppe mit einem C₁-C₃-Alkohol verethert sein kann, ist.

12. Formulierung gemäß Anspruch 1 oder 2, wobei der organische Triazol-UV-Absorber die Formel 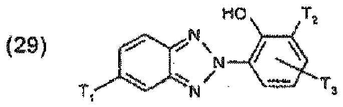 aufweist, wobei
T₁ C₁-C₃-Alkyl oder, vorzugsweise, Wasserstoff; oder ein Rest der Formel 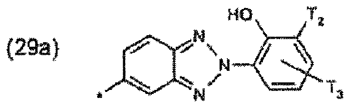 ist; und
T₂ und T₃ unabhängig voneinander C₁-C₁₂-Alkyl, vorzugsweise i-Octyl; oder C₁-C₄-Alkyl, substituiert mit Phenyl, vorzugsweise α,α-Dimethylbenzyl, sind.

13. Formulierung gemäß Anspruch 2, wobei der organische Benzotriazol-UV-Absorber der Formel 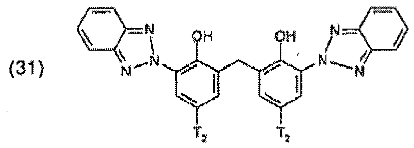 entspricht, wobei
T₂ Wasserstoff; C₁-C₁₂-Alkyl; oder C₁-C₄-Alkyl, substituiert mit Phenyl, ist.

14. Formulierung gemäß Anspruch 13, wobei T₂ iso-Octyl ist.

15. Formulierung gemäß einem der vorstehenden Ansprüche, wobei die Sonnenschutzformulierung zusätzlich einen öllöslichen organischen UV-Absorber umfasst, ausgewählt aus einem p-Aminobenzoesäurederivat; einem Salicylsäurederivat; einem Benzophenonderivat; einem Dibenzoylmethanderivat; einem Diphenylacrylatderivat; einem Benzofuranderivat; einem polymeren UV-Absorber, der einen oder mehrere silico-organischen Reste enthält; einem Cinnamatester; einem Campherderivat; einem Trianilino-s-triazinderivat; Phenylbenzimidazolsulfonsäure oder einem ihrer Salze; Urocansäure (3-Imidazol-4-ylacrylsäure) oder ihrem Ethylester; Menthylanthranilat; einem Benzotriazol; einem Hydroxyphenyltriazinderivat; und einem Bisresorcinoldialkylaminotriazin.

## Revendications

1. Formulation eau dans huile (w/o) ou eau dans silicone (w/Si) à appliquer en topique, comprenant:
(a) 10 à 90 % en poids d'au moins une phase aqueuse;
(b) 1 à 60 % en poids d'au moins une phase grasse ;
(c) 0,1 à 30 % en poids d'une composition photoprotectrice contre les UV comprenant
(C₁) 20% à 99% d'au moins un actif anti-UV organique micronisé insoluble ayant une granulométrie moyenne s'échelonnant de 0,01 µm à 2 µm;
(C₂) 0,5% à 80% d'un mélange d'octanol linéaire et de décanol linéaire ;
0 à 30 % en poids d'au moins un émulsifiant ; et
0 à 88,9 % en poids d'autres adjuvants acceptables en cosmétique.

2. Formulation selon la revendication 1, dans laquelle
(C₁) est choisi parmi une triazine, un benzotriazole, une benzophénone, un amide contenant un groupe vinyle, un amide d'acide cinnamique et un absorbeur UV de type benzimidazole sulfoné.

3. Formulation selon la revendication 1 ou 2, dans laquelle l'absorbeur UV de type triazine répond à la formule 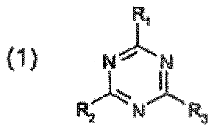 dans laquelle
R₁, R₂ et R₃, indépendamment les uns des autres, représentent des atomes d'hydrogène ; des groupes hydroxy ; alcoxy en C₁-C₃ ; NH₂; NHR₄ ; N (R₄)₂; OR₄; aryle en C₆-C₁₂ ; phénoxy; anilino; pyrrolo ; dans lesquels les groupements phényle, phénoxy, anilino ou pyrrolo respectifs ne sont pas substitués ou sont substitués par un, deux ou trois substituants choisis parmi OH, carboxy, CO-NH₂, alkyle en C₁-C₁₂, alcoxy en C₁-C₁₂, un groupe méthylidènecamphre, un groupe - (CH=CH)ₘC(=O)-OR₄, un groupe 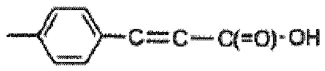 ou les sels correspondants de métal alcalin, d'ammonium, de mono-, di- ou tri-alkyl-(en C₁-C₄) ammonium, de mono-, di- ou tri-alcanol(en C₂-C₄) ammonium, ou leurs esters d'alkyle en C₁-C₃, ou par
un radical de formule 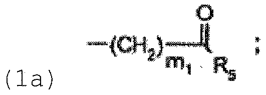 R₄ représente un groupe alkyle en C₁-C₅;
R₅ représente un groupe hydroxy ; alkyle en C₁-C₅ qui est non substitué ou substitué par un ou plusieurs groupes OH ; alcoxy en C₁-C₅ ; amino ; mono- ou dialkyl(en C₁-C₅) amino ; M ; un radical de formule 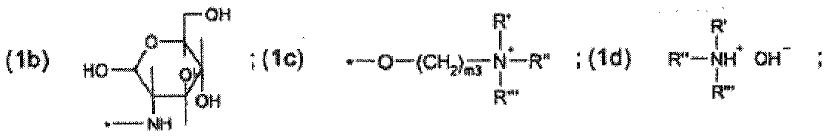 ou 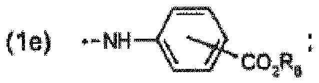 R', R" et R"', indépendamment les uns des autres, représentent un groupe alkyle en C₁-C₁₄ qui est non substitué ou substitué par un ou plusieurs groupes OH ;
R₆ représente un atome d'hydrogène ; M ; un groupe alkyle en C₁-C₅ ; ou un radical de formule -(CH₂)ₘ₂-O-T₁ ;
M représente un cation métallique ;
T₁ représente un atome d'hydrogène ; ou un groupe alkyle en C₁-C₈ ,
m vaut 0 ou 1 ;
m₁ vaut de 1 à 5 ;
m₂ vaut de 1 à 4 ; et
m₃ vaut de 2 à 14.

4. Formulation selon la revendication 1 ou 2, dans laquelle l'absorbeur UV de type triazine répond à la formule (I), dans laquelle
R₁, R₂ et R₃, indépendamment les uns des autres, représentent un radical de formule 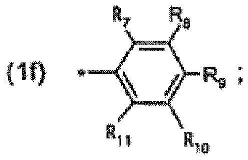 ou 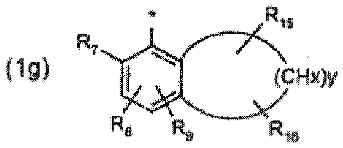 R₇ et R₁₁, indépendamment les uns des autres, représentent un atome d'hydrogène ; un groupe alkyle en C₁-C₁₈ ; ou aryle en C₆-C₁₂
R₈, R₉ et R₁₀, indépendamment les uns des autres, représentent un atome d'hydrogène ; ou un radical de formule 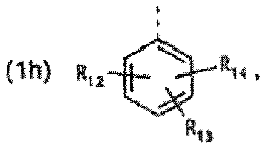 dans laquelle, dans la formule (1f), au moins un des radicaux R₈, R₉ et R₁₀ représentent un radical de formule (1h)
R₁₂, R₁₃, R₁₄, R₁₅ et R₁₆, indépendamment les uns des autres, représentent des atomes d'hydrogène ; des groupes hydroxy ; halogéno ; alkyle en C₁-C₁₈ ; alcoxy en C₁-C₁₈ ; aryle en C₆-C₁₂ ; biphénylyle ; aryloxy en C₆-C₁₂ ;
alkylthio en C₁-C₁₈ ; carboxy ; -COOM ; alkyl (en C₁-C₁₈) - carboxyle ; aminocarbonyle ; mono- ou di-alkyl(en C₁-C₁₈) amino ; acylamino en C₁-C₁₀ ; ou -COOH ;
M représente un ion de métal alcalin ;
x vaut 1 ou 2 ; et
y est un nombre de 2 à 10.

5. Formulation selon la revendication 4, dans laquelle les triazines répondent à la formule 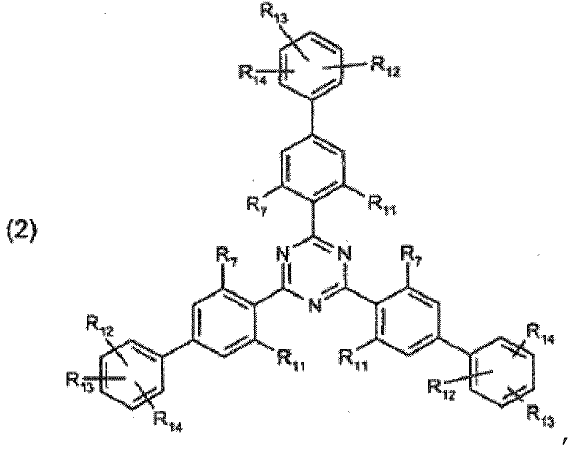 dans laquelle R₇, R₁₁, R₁₂, R₁₃ et R₁₄ sont définis selon la revendication 4.

6. Formulation selon la revendication 5, dans laquelle R₇ et R₁₁ représentent un atome d'hydrogène.

7. Formulation selon la revendication 4, dans laquelle le constituant (C₁) désigne les composés de formule 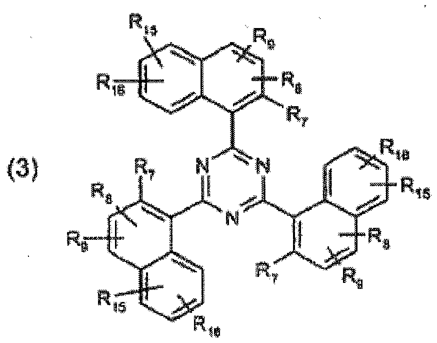 dans laquelle
R₇, R₈, R₉, R₁₅ et R₁₆ sont définis selon la revendication 4.

8. Formulation selon la revendication 7, dans laquelle R₇, R₈, R₉, R₁₅ et R₁₆ représentent des atomes d'hydrogène ou, indépendamment les uns des autres, des groupes alkyle en C₁-C₁₈.

9. Formulation selon la revendication 5, dans laquelle le constituant (C₁) répond à la formule

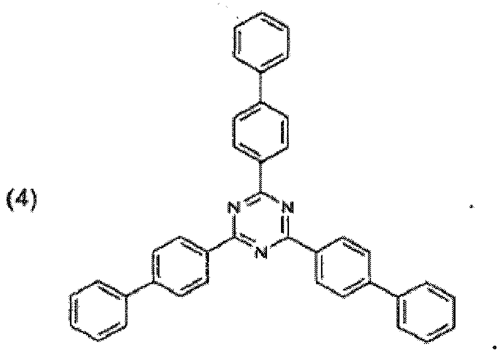

10. Formulation selon l'une quelconque des revendications 1 à 3, dans laquelle l'absorbeur UV de type triazine a pour formule 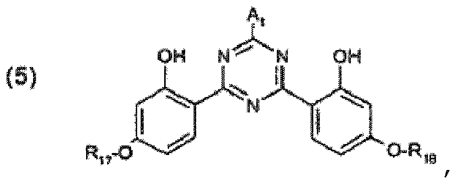 dans laquelle
R₁₇ et R₁₈, indépendamment l'un de l'autre, représentent un groupe alkyle en C₁-C₁₈ ; un groupe alcényle en C₂-C₁₈ ; un radical de formule -CH₂-CH(-OH)-CH₂-O-T₁ ; ou un radical de formule -(CH₂)ₘ₁-O-(CH₂)ₘ₂-T₂ ; un radical de formule 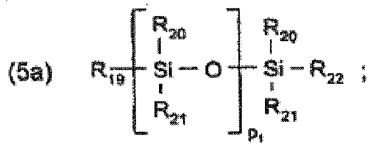 R₁₉ représente une liaison directe ; un radical alkylène en C₁-C₄ à chaîne droite ou ramifiée ou un radical de formule -Cₘ₁H₂ₘ₁- ou -Cₘ₁H₂ₘ₁-O- ;
R₂₀, R₂₁ et R₂₂, indépendamment les uns des autres, représentent un groupe alkyle en C₁-C,₈ ; alcoxy en C₁-C₁₈ ou
un radical de formule 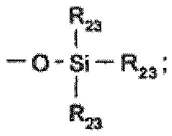 R₂₃ représente un groupe alkyle en C₁-C₅ ;
T₁ et T₂, indépendamment l'un de l'autre, représentent des atomes d'hydrogène ; ou des groupes alkyle en C₁-C₈ ;
m₁, m₂ et m₃, indépendamment les uns des autres, valent 1 à 4 ;
p₁ vaut 0 ; ou un nombre de 1 à 5 ;
A₁ représente un radical de formule 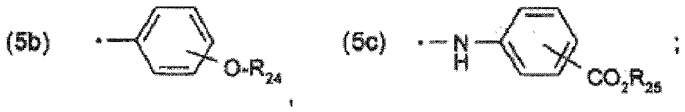 ou de formule 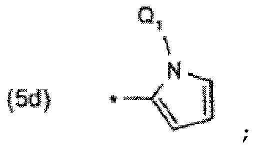 R₂₄ représente un atome d'hydrogène ; un groupe alkyle en C₁-C₁₀, -(CH₂CHR₂₆-O)ₙ₁-R₂₅ ; -CH₂-CH (-OH) -CH₂-O-T₁ ; ou un radical de formule -(CH₂)ₘ₁-O-(CH₂)ₘ₂-T₂ ;
R₂₅ représente un atome d'hydrogène ; M ; un groupe alkyle en C₁-C₅ ; ou un radical de formule - (CH₂) ₘ₂O-T₁ ; R₂₆ représente un atome d'hydrogène ou un groupe méthyle ;
Q1 représente un groupe alkyle en C₁-C₁₈ ;
M représente un cation métallique ; et
n₁ vaut 1-16.

11. Formulation d'écran solaire selon la revendication 1 ou 2 dans laquelle le composé triazine selon le constituant (C₁) répond à la formule 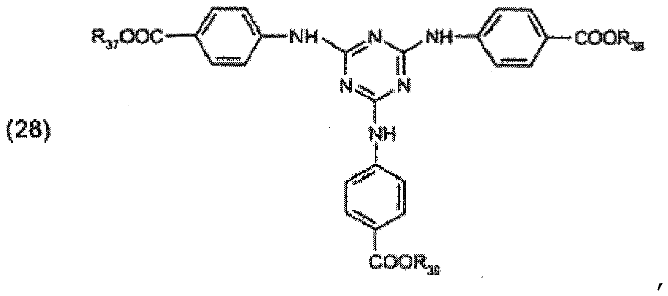 dans laquelle
R₃₇, R₃₈ et R₃₉, indépendamment les uns des autres, représentent un atome d'hydrogène; un métal alcalin ; un groupe ammonium N⁺ (P₄₀)₄ ;
R₄₀ représente un atome d'hydrogène ; ou un radical organique; un groupe alkyle en C₁-C₃; ou un radical polyoxyéthylène qui contient de 1 à 10 motifs oxyde d'éthylène et dont le groupe OH terminal peut être éthérifié par un alcool en C₁-C₃.

12. Formulation selon la revendication 1 ou 2 dans laquelle l'absorbeur UV organique de type triazole a pour formule 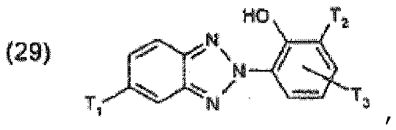 dans laquelle
T₁ représente un groupe alkyle en C₁-C₃ ou, de préférence, un atome d'hydrogène ; ou un radical de formule 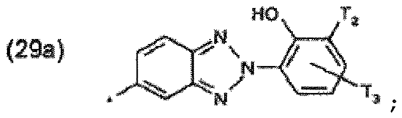 et
T₂ et T₃, indépendamment l'un de l'autre, représentent un groupe alkyle en C₁-C₁₂, de préférence i-octyle ; ou alkyle en C₁-C₄ substitué par un phényle, de préférence α,α-diméthylbenzyle.

13. Formulation selon la revendication 2 dans laquelle l'absorbeur UV organique de type benzotriazole répond à la formule 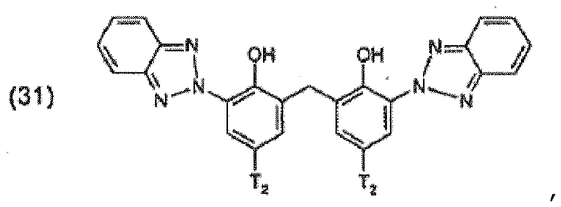 dans laquelle T₂ représente un atome d'hydrogène ; un groupe alkyle en C₁-C₁₂ ; ou alkyle en C₁-C₄ substitué par un phényle.

14. Formulation selon la revendication 13 dans laquelle T₂ représente un groupe iso-octyle.

15. Formulation selon l'une quelconque des revendications précédentes dans laquelle la formulation d'écran solaire comprend en plus un absorbeur UV organique oléosoluble choisi parmi un dérivé d'acide p-amino-benzoïque; un dérivé d'acide salicylique ; un dérivé de benzophénone ; un dérivé de dibenzoylméthane ; un dérivé d'acrylate de diphényle ; un dérivé de benzofurane ; un absorbeur UV polymère contenant un ou plusieurs résidus silico-organiques ; un ester cinnamate ; un dérivé de camphre ; un dérivé de trianilino-s-triazine ; l'acide phénylbenzimidazolesulfonique ou un de ses sels ; l'acide urocanique (acide 3-imidazol-4-yl-acrylique) ou son ester éthylique ; l'anthranilate de menthyle ; un benzotriazole ; un dérivé d'hydroxyphényltriazine ; et une bis-résorcinoldialkylaminotriazine.
